# EUROPÄISCHE PATENTSCHRIFT

(11) **EP 2 776 551 B1**
(45) Veröffentlichungstag und Bekanntmachung des Hinweises auf die Patenterteilung: **26.08.2015**
(21) Anmeldenummer: 12786972.5
(22) Anmeldetag: 09.11.2012
(51) Int. Cl.: C12M 1/107

(54) **EINBRINGSCHNECKE FÜR BIOGASANLAGEN**
INTRODUCING SCREW FOR BIOGAS PLANTS
VIS SANS FIN D'ALIMENTATION POUR INSTALLATIONS À BIOGAZ

(30) Priorität: 11.11.2011 DE 202011107750 U
(43) Veröffentlichungstag der Anmeldung: 17.09.2014
(73) Patentinhaber: Hugo Vogelsang Maschinenbau GmbH, 49632 Essen (DE)
(72) Erfinder: VOGELSANG, Hugo, 49632 Essen (DE)
(74) Vertreter: Eisenführ Speiser
(86) Internationale Anmeldenummer: PCT/EP2012/072313
(87) Internationale Veröffentlichungsnummer: WO 2013/068555

(56) Entgegenhaltungen:
- DE-A1- 10 252 527
- DE-U1- 20 216 090
- DE-U1- 29 903 208
- DE-U1-202009 003 782
- DE-U1-202010 000 550
- US-A1- 2009 022 570

## Beschreibung

Die Erfindung betrifft einen Schneckenförderer zum Einbringen organischer Stoffe in einen unter Gasinnendruck stehenden Biogasfermenter. Ein weiterer Aspekt der Erfindung ist ein Verfahren zum Beschicken einer solchen Biogasanlage mit organischen Feststoffen.

In Biogasanlagen wird durch Vergärung organischer Stoffe in einem Fermenter ein brennbares Gas, typischerweise durch Methanogenese, erzeugt. Die Fermenter werden dabei typischerweise unter einem Gasinnendruck betrieben, der durch gasdichten Verschluss des Fermenter und kontrollierten Abzug des darin erzeugten Biogases erzeugt wird. Fermenter können kontinuierlich oder quasi-kontinuierlich (batch-weise) mit organischen Feststoffen beschickt werden, um den Gärungsprozess in Gang zu halten. Sowohl bei der Beschickung als auch danach muss der Innendruck im Fermenter überwunden bzw. aufrechterhalten und ein unerwünschter Austritt von Stoffen aus dem Fermenter verhindert werden.

Aus DE 202 16 090 U1 ist eine Vorrichtung zum Einbringen von Stallabfällen in einen Güllefermenter bekannt, bei der mittels mehrerer Schneckenförderer die Stallabfälle von oben in den Fermenter eingefüllt werden. Aus DE 299 03 208 ist eine ähnliche Vorrichtung zur Beschickung eines Gärbehälters einer Biogasanlage bekannt.

Neben den so vorbekannten Vorrichtungen zur Beschickung eines Gärbehälters von oben sind auch Vorrichtungen zur Beschickung des Gärbehälters von unten bekannt, die häufig eine bessere Durchmischung des zugeführten Feststoffs mit dem bereits im Behälter befindlichen Feststoff erzielen.

Eine grundsätzliche Problematik bei der Zufuhr der Feststoffe in den Fermenter besteht darin, dass ein Rückstrom von Feststoffen, Flüssigkeiten oder Gasen aus dem Fermenter durch die Zufuhrvorrichtung sicher verhindert werden muss. Diese Verhinderung muss sowohl dann erreicht werden, wenn die Zufuhrvorrichtung in Betrieb ist als auch dann, wenn die Zufuhrvorrichtung nicht in Betrieb ist, wie beispielsweise bei der quasikontinuierlichen Zufuhr von Feststoffen in zeitlich gestaffelten, aufeinanderfolgenden Zufuhrvorgängen.

Aus DE 102 52 527 B4 ist eine Fördervorrichtung zum Einbringen von organischen Feststoffen in einen Vergärungsbehälter bekannt, welche einen Schneckenförderer umfasst, der einen schneckenflügellosen Abschnitt aufweist. Mit dieser Vorrichtung wird bezweckt, im Bereich des schneckenflügellosen Abschnitts eine Verpressung des Förderguts zu erzielen, um hierdurch eine Abdichtung der Förderschnecke gegen Rückstrom von Feststoffen, Flüssigkeiten und Gas zu erreichen. Durch den schneckenflügellosen Abschnitt im Schneckenförderer ist die Förderleistung des Schneckenförderer jedoch herabgesetzt und bei schwierig zu fördernden Feststoffen kann der innere Widerstand im Schneckenförderer soweit ansteigen, dass die Förderleistung auf Null abfällt, folglich eine Verstopfung auftritt.

Aus DE 20 2010 000 550 U1 ist eine demgegenüber weiterentwickelte Zuführvorrichtung für Feststoff in einen Gärbehälter bekannt, die ebenfalls einen schneckenflügellosen Abschnitt aufweist und weiterhin mit einer axial beweglichen Schnecke ausgerüstet ist. Durch die axiale Beweglichkeit sollen Verstopfungen des Schneckenförderers verhindert werden. Die Vorrichtung hat sich diesbezüglich zwar als vorteilhaft erwiesen, erfordert aber eine aufwendige und fehleranfällige Lagerung und Betätigung der Förderschnecke, um die überlagerte axiale Beweglichkeit und Rotation zu bewerkstelligen.

Der Erfindung liegt die Aufgabe zugrunde, eine Zuführvorrichtung für organische Feststoffe in Biogasanlagen bereitzustellen, welche eine zuverlässige Förderung der organischen Feststoffe erreicht und zugleich eine zuverlässige Abdichtung des Biogasbehälters gegen Austritt von Feststoffen, Flüssigkeiten oder Gasen durch die Zuführvorrichtung im Betrieb und im Stillstand der Zuführvorrichtung gewährleistet.

Diese Aufgabe wird erfindungsgemäß gelöst durch einen Schneckenförderer der eingangs genannten Art, bei dem der Stopfabschnitt einen Erweiterungsbereich aufweist, in dem sich der Durchtrittsquerschnitt zwischen der Förderschnecke und dem Schneckenrohr in Förderrichtung erweitert und einen in Förderrichtung hinter dem Erweiterungsbereich liegenden Verengungsbereich aufweist, in dem sich der Durchtrittsquerschnitt zwischen der Förderschnecke und dem Schneckenrohr in Förderrichtung reduziert.

Mit der so definierten Schneckenfördervorrichtung wird eine effiziente und zugleich zuverlässige Zufuhr von organischen Feststoffen in einen unter Druck stehenden Fermenter ermöglicht, ohne dass hierbei in jeglicher Zufuhranordnung und unter jeglichem Betriebszustand die Gefahr besteht, dass Feststoffe, Flüssigkeiten oder Gase durch den Schneckenförderer aus dem Fermenter austreten. Dies wird erreicht, indem ein Stopfabschnitt im Schneckenförderer vorgesehen ist, an dem die organischen Feststoffe in solcher Weise wirksam verdichtet werden, dass sie eine zuverlässige Sperre gegen einen solchen Durchtritt von Feststoffen, Flüssigkeiten oder Gasen bilden. Zugleich ist dieser Stopfabschnitt mittels eines darin ausgebildeten Schneckenflügels so beschaffen, dass ein Stillstand durch eine Verstopfung zuverlässig vermieden werden kann und eine zuverlässige Förderung bei Betrieb des Schneckenförderers erfolgt. Der Stopfabschnitt zeichnet sich dabei dadurch aus, dass sich sein Durchtrittsquerschnitt zunächst erweitert und hierauf folgend in Förderrichtung wieder verengt, wodurch die gewünschte Verdichtung und Abdichtung erzielt wird.

Unter dem Durchtrittsquerschnitt soll hierbei der Raum verstanden werden, der innerhalb des Schneckenrohrs als Querschnitt vorhanden ist und nicht durch in dem Schneckenrohr eingebaute feststehende oder bewegliche Elemente, wie insbesondere die Förderschnecke mit den darauf angeordneten Schneckenflügeln ausgefüllt wird. Bei typischen Schneckenförderern wird der Durchtrittsquerschnitt daher als ringförmiger Spalt ausgebildet, der zwischen dem Schneckenwellenkern und der Innenwandung des Schneckenrohrs ausgebildet ist und je nach Anzahl der Schneckenflügel partiell durch einen oder mehrere Querschnitte der Schneckenflügel reduziert wird. Die Erweiterung dieses Durchtrittsquerschnitt kann einerseits durch eine Reduktion der Querschnitte der Bauteile innerhalb des Schneckenrohrs erfolgen, beispielsweise eine Reduktion des Durchmessers des Schneckenwellenkerns oder durch eine Vergrößerung des Schneckenrohrinnendurchmessers oder durch beide Maßnahmen. Dem gegenüber kann die Verringerung des Durchtrittsquerschnitts durch eine Vergrößerung der Querschnitte der eingebauten Bauteile innerhalb des Schneckenrohres erfolgen, beispielsweise eine Durchmesservergrößerung des Kerns der Schneckenwelle oder eine Verdickung des Querschnitts der Schneckenflügel und kann alternativ oder zusätzlich durch eine Reduktion des Innendurchmessers des Schneckenrohrs erfolgen.

Zur Erzielung einer wirksamen Verdichtung ist es erfindungsgemäß vorgesehen, dass im Stopfabschnitt zunächst eine Erweiterung des Durchtrittsquerschnitts stattfindet, die in Förderrichtung im vorderen Bereich des Stopfabschnitts erfolgt. In dem in Förderrichtung hieran anschließenden Bereich; also dem in axialer Richtung größten Teil des Stopfabschnitts, der den mittleren und den Endbereich des Stopfabschnitts vorzugsweise umfasst, erfolgt eine Durchtrittsquerschnittsverringerung. Grundsätzlich ist zu verstehen, dass sowohl die Erweiterung als auch die Verringerung des Durchtrittsquerschnitts in einer oder mehrerer Stufen, also diskontinuierlich erfolgen kann und alternativ oder in Kombination hierzu auch vollständig oder abschnittsweise kontinuierlich erfolgen kann. Unter einer kontinuierlichen Änderung eines Durchtrittsquerschnitts ist hierbei beispielsweise eine konstant zunehmende oder abnehmende Querschnittsänderung zu verstehen, ebenso aber auch eine progressiv oder degressiv sich ändernde Querschnittsverringerung.

Gemäß einer ersten bevorzugten Ausführungsform ist vorgesehen, dass die Förderschnecke in einem Bereich unmittelbar in Förderrichtung zu Beginn des Stopfabschnitts einen ersten Durchtrittsquerschnitt zwischen der Förderschnecke und dem Schneckenrohr aufweist, hinter dem ersten Durchtrittsquerschnitt im Anfangsbereich des Stopfabschnitts einen zweiten, gegenüber dem ersten Durchtrittsquerschnitt vergrößerten Durchtrittsquerschnitt aufweist und am Ende des Stopfabschnitts einen dritten, gegenüber dem zweiten verringerten Durchtrittsquerschnitt aufweist. Mit dieser Ausgestaltung wird eine zunächst über eine kurze axiale Erstreckung erfolgende Erweiterung des Durchtrittsquerschnitts, gefolgt von einer über einen längeren axialen Abschnitt des Stopfabschnitts erfolgende Verringerung des Durchtrittsquerschnitts definiert, die sich als besonders vorteilhaft hinsichtlich einer effizienten Verdichtung und sicheren Förderung erwiesen hat.

Weiter ist es bevorzugt, dass sich der Durchtrittsquerschnitt im Stopfabschnitt in Förderrichtung im Anfangsbereich stufig erweitert und/oder über den Großteil des Stopfabschnitts kontinuierlich verringert. Durch die so definierte stufige Erweiterung, gefolgt von einer kontinuierlichen Verringerung wird ein Verstopfen des Schneckenförderers zuverlässig verhindert und zugleich eine ausreichende Verdichtung der Feststoffe im Stopfabschnitt erzielt, um eine ausreichende Abdichtung gegen einen Gasdurchtritt zu erzielen.

Gemäß einer weiteren bevorzugten Ausführungsform ist vorgesehen, dass sich der Durchtrittsquerschnitt vor dem Stopfabschnitt ausgehend von einem ersten Durchtrittsquerschnitt, der eine durch ein erstes Kugeldurchgangsmaß gekennzeichnete Querschnittsgeometrie aufweist, auf einen zweiten Durchtrittsquerschnitt mit einer Querschnittsgeometrie, die durch zweites Kugeldurchgangsmaß gekennzeichnet ist, das größer als das erste Kugeldurchgangsmaß ist, ändert und weiter auf einen dritten Durchtrittsquerschnitt mit einer Querschnittsgeometrie, die durch ein drittes Kugeldurchgangsmaß gekennzeichnet ist, das kleiner als das zweite Kugeldurchgangsmaß ist, ändert, wobei das erste und das dritte Kugeldurchgangsmaß vorzugsweise übereinstimmend sind. Unter einem Kugeldurchgangsmaß ist hierbei der Durchmesser zu verstehen, den eine Kugel maximal aufweisen darf, um durch eine entsprechende Stelle, die durch das Kugeldurchgangsmaß gekennzeichnet ist, im Schneckenförderer gefördert zu werden. Das Kugeldurchgangsmaß ist in der Anwendung zur Förderung organischer Feststoffe in Biogasanlagen insbesondere daher relevant, weil hierdurch eine Charakterisierung der Fähigkeit des Schneckenförderers erfolgen kann, inwieweit dieser in der Lage ist, in den organischen Feststoffen enthaltene Hartstoffe wie insbesondere Steine zu fördern, ohne hierbei beschädigt zu werden oder zu blockieren. Je höher das Kugeldurchgangsmaß ist, desto unempfindlicher und leistungsfähiger ist der Schneckenförderer in dieser Eigenschaft. Mit der erfindungsgemäßen Ausgestaltung wird insbesondere erreicht, dass eine Verdichtung der organischen Feststoffe durch entsprechende Durchtrittsquerschnittsverringerungen erreicht wird, ohne dass hierbei notwendiger Weise das Kugeldurchtrittsmaß über die gesamte Länge des Schneckenförderers ausgehend von einem anfänglichen Kugeldurchgangsmaß unter dieses Maß fallen muss.

Gemäß eines weiteren vorteilhaften Aspekts der Erfindung wird ein Schneckenförderer der Eingangs beschriebenen Art bereitgestellt, welcher sich durch einen in Förderrichtung vor dem Stopfabschnitt angeordneten Separationsabschnitt, umfassend eine Mehrzahl von Öffnungen im Schneckenrohr und eine Flüssigkeitsauffangvorrichtung außerhalb des Schneckenrohres auszeichnet. Der Schneckenförderer kann insbesondere ausgebildet sein nach einer der zuvor erläuterten erfindungsgemäßen Ausführungen. Durch den gemäß dieser Ausgestaltung bereitgestellten Separationsabschnitt wird verhindert, dass sich der Inhalt der Schneckenförderers mit Flüssigkeit anreichert und auf diese Weise eine zu niedrige Viskosität erreichen könnte, um noch wirksam mit dem Schneckenförderer gefördert zu werden. Die Entwässerung kann hierbei insbesondere durch eine Vielzahl von über den Umfang verteilten Öffnungen entlang der axialen Erstreckung des Separationsabschnitts erfolgen, beispielsweise durch eine siebähnliche Ausführung des Schneckenrohrs im Bereich des Separationsabschnitts. Durch die Anordnung des Separationsabschnitts in Förderrichtung vor dem Stopfabschnitt wird eine wirksame Verdichtung der organischen Feststoffe im Stopfabschnitt zusätzlich unterstützt.

Ein weiterer Aspekt der Erfindung ist ein Schneckenförderer der Eingangs beschriebenen Art, der sich durch eine Spülflüssigkeitszufuhröffnung zum Zuführen einer Spülflüssigkeit in den Durchtrittsquerschnitt zwischen Schneckenrohr und Förderschnecke im Bereich des zweiten Schneckenrohrendes in Förderrichtung hinter dem Stopfabschnitt auszeichnet. Eine solche Spüleinlassöffnung ermöglicht es, dass im Bereich der Austrittsöffnung am zweiten Schneckenrohrende die verdichtete und ggfs. flüssigkeitsreduzierte organische Masse zuverlässig aus dem Schneckenförderer herausgefördert wird, indem diese zusätzlich verflüssigt wird, d. h. eine Viskositätsverringerung erfährt. Zur Spülung kann hierbei ein Trägermedium wie beispielsweise Rezirkulat oder Gülle, insbesondere eine im Bereich eines Separationsabschnitts gewonnene Flüssigkeit verwendet werden. Das Einspülen der Spülflüssigkeit fördert zudem die Durchmischung des zuvor erzeugten, verdichteten Stopfens, was für die spätere Fermentation eine günstige Ausgangslage erzeugt.

Dabei ist bevorzugt vorgesehen, dass eine Spüleinlassöffnung an der Stirnseite des Schneckenrohres angeordnet ist und in eine axial in Längsrichtung des Schneckenrohres verlaufende Spülleitung mündet, die sich ausgehend vom zweiten Ende des Schneckenrohres zu mindestens einer radialen Spülflüssigkeitszufuhröffnung erstreckt, die in den Durchtrittsquerschnitt zwischen Förderschnecke und Schneckenrohr mündet. Mit dieser Ausgestaltung wird eine vorteilhafte Konstruktion für eine Einleitung der Spülflüssigkeit im Bereich des zweiten Endes des Schneckenförderers bereitgestellt, die insbesondere in Zusammenwirkung mit einer radialen Auslassöffnung aus dem Schneckenrohr eine wartungsfreundliche und zugleich robuste Konstruktion zulässt. Vorteilhaft sind mehrere, über den Umfang verteilte radiale Spülflüssigkeitszufuhröffnungen vorgesehen, die sich aus dem Innenraum der Schneckenwelle oder der Spülleitung erstrecken, um eine zuverlässige Durchmischung der organischen Feststoffe mit der Spülflüssigkeit im Bereich des zweiten Endes zu erreichen.

Gemäß einer weiteren Fortbildungsform hierzu ist vorgesehen, dass die Förderschnecke im Bereich des zweiten Endes als Hohlwelle ausgebildet ist zur Leitung der Spülflüssigkeit im Innenraum der Förderschnecke. Durch diese Ausgestaltung wird eine unmittelbare Zuförderung der Spülflüssigkeit in den Bereich der organischen Feststoffe mit einer wirksamen Durchmischung ermöglicht.

Noch weiter ist es bevorzugt, dass die Förderschnecke im Bereich des zweiten Endes auf einem Stutzen drehbar gelagert ist, der drehmomentfest am Schneckenrohr befestigt ist und sich axial in einen Hohlraum der Förderschnecke erstreckt. Mit dieser Ausführungsform wird einerseits eine wirksame Spülung und Durchmischung im Bereich des zweiten Endes erzielt, zugleich aber auch eine montagetechnisch einfache und robuste Lagerung der Förderschnecke ermöglicht. Der Stutzen kann dabei insbesondere zur Leitung der Spülflüssigkeit genutzt werden.

Gemäß einer weiteren bevorzugten erfindungsgemäßen Ausführung kann der Eingangs erläuterte Schneckenförderer fortgebildet werden durch zumindest einen Schneckenflügel im Bereich des zweiten Schneckenrohrendes, der lösbar an der Förderschnecke befestigt ist. Grundsätzlich können im Bereich des zweiten Endes Schneckenflügel angeordnet sein, die sich gewindeförmig um die Schneckenwelle herum legen oder die nach Art von Schaufeln sich axial in Längsrichtung an der Schneckenwelle erstrecken. Beide Ausgestaltungen von Schneckenflügeln können insbesondere dazu genutzt werden, um die ggfs. durch eine Spülflüssigkeit verflüssigten organischen Feststoffe aus einer radial angeordneten Auslassöffnung im Schneckenrohr hinauszufördern. Diese Schneckenflügel im Bereich des zweiten Endes können dabei insbesondere als konstruktive Steuergrößen eingesetzt werden, in dem sie den Durchtrittsquerschnitt an diesem zweiten Ende in einem geringen oder einem größeren Maße durch ihre Geometrie verringern. So kann es bei Förderung von organischen Feststoffen mit hohem Flüssigkeitsgehalt vorteilhaft sein, wenn im Bereich des zweiten Endes Schneckenflügel montiert werden, die eine hohe Querschnittsverringerung in diesem Endbereich bewirken, um hierdurch die Verdichtungswirkung im Stopfabschnitt zu unterstützen. Im umgekehrten Fall, bei Förderung besonders trockener organischer Feststoffe, ist eine möglichst geringe Querschnittsverringerung im Bereich des zweiten Endes wünschenswerts und es können entsprechend schmale Schneckenflügel montiert werden. Um die erfindungsgemäße Schneckenfördervorrichtung auf einfache Weise an unterschiedliche Konsistenzen der organischen Feststoffe anpassen zu können und in allen Fällen eine ideale Verdichtung und Förderung zu erzielen, ist es daher vorteilhaft, wenn die Schneckenflügel im Bereich des zweiten Endes lösbar montiert sind. Dabei ist zu verstehen, dass sich diese Schneckenflügel im Bereich des zweiten Endes als eine Verlängerung des Schneckenflügel der Schneckenwelle selbst ausgeführt sein können und dementsprechend nur als lösbar montierter Abschnitt ausgeführt sind oder die Schneckenflügel sind als separates und in der Steigung ggfs. unterschiedliches Bauelement zu dem/der Schneckenflügel im übrigen Bereich der Förderschnecke ausgebildet. Grundsätzlich kann ein einziger Schneckenflügel oder mehrere Schneckenflügel im Bereich des zweiten Endes angeordnet sein und im Falle von mehreren Schneckenflügeln können von diesen einer oder mehrere lösbar montiert sein.

Dabei ist weiter bevorzugt vorgesehen, dass der erfindungsgemäße Schneckenförderer fortgebildet wird durch einen ersten auf dem Schneckenförderer montierbaren Schneckenflügel und einen zweiten, alternativ zum ersten montierbaren Schneckenflügel; der einen Querschnitt aufweist, der gegenüber dem ersten Schneckenflügel eine größere Querschnittsfläche des Durchtrittsquerschnitts zwischen Förderschnecke und Schneckenrohr ausfüllt. Mit dieser Ausgestaltung wird ein Bausatz an unterschiedlichen Schneckenflügeln für den Bereich des zweiten Endes des Schneckenförderers bereitgestellt, der eine Anpassung an unterschiedliche organische Feststoffe oder Förderaufgaben ermöglicht.

Ein weitere Aspekt der Erfindung ist ein Schneckenförderer der eingangs beschriebenen Bauweise, der fortgebildet ist, indem die Innenwandung des Schneckenrohrs eine nicht-kreisrunde innere Querschnittsfläche umschließt. Diese erfindungsgemäße Ausgestaltung löst ein spezifisches Problem von Schneckenförderern, die mit unterschiedlichen Feststoffen betrieben werden und welches darin liegt, dass solche Schneckenförderer bei bestimmten Konsistenzen der organischen Feststoffe eine Verringerung ihrer Förderwirkung erfahren, weil sich das Fördergut teilweise in dem Förderrohr mitdreht. Sobald ein solches teilweises oder sogar vollständiges mitdrehen des Fördergutes mit der Förderschnecke auftritt, ist die Förderleistung signifikant reduziert oder entfällt sogar vollständig. Durch eine nicht kreisrunde Ausführung des durch den die Innenwandung des Schneckenrohrs umschlossenen Querschnitts wird ein mitdrehen des Förderguts zuverlässig verhindert, indem sich das Fördergut an entsprechend nicht kreisrunden. Geometriebereichen der Rohrwandungdrehmoment abstützen kann und folglich die Haftung zum Rohr erhöht wird. Unter einer nichtkreisrunden inneren Querschnittsfläche soll hierbei jegliche Querschnittsfläche verstanden werden, die nicht durch einen Kreis begrenzt wird, dies umfasst insbesondere ovale Querschnittsbegrenzungen, unregelmäßige Querschnittsbegrenzungen wie beispielsweise nach einer dreifach oder vierfach elipsoiden Geometrie.

Insbesondere ist es bevorzugt, dass die Innenwandung des Schneckenrohrs eine eckige innere Querschnittsfläche umschließt. Die solcherart eckige innere Querschnittsfläche kann beispielsweise durch eine dreieckige Kontur außen begrenzt werden, ebenso sind Begrenzungen durch vier-, fünf-, sechseckige Außenkonturen in bestimmten Anwendungen vorteilhaft und weiterhin können auch Außenkonturbegrenzungen durch mehreckige Formen mit mehr als sechs Ecken vorteilhaft sein. Dabei ist zu verstehen, dass die Wahl des Grades der Abweichung vom kreisrunden Querschnitt einerseits maßgeblich ist für die Erhöhung der Haftung der Feststoffe an der Rohrinnenwandung, andererseits maßgeblich dafür ist, wie viel Spaltmaß zwischen den Schneckenflügeln und dem Schneckenrohr auftritt, was zu einer Leckagewirkung führen kann. Je näher die Querschnittsgeometrie der kreisrunden Form angenähert wird, desto geringer kann dieses Spaltmaß gewählt werden, desto geringer ist aber auch die zusätzliche Haftungswirkung durch die nicht-kreisförmige Geometrie und folglich desto höher ist die Gefahr einer Mitrotation der Feststoffe mit der Förderschnecke.

Gemäß eines weiteren Aspekts der Erfindung wird ein Schneckenförderer bereitgestellt, umfassend ein sich in einer Längsrichtung erstreckendes Schneckenrohr mit einer Einlassöffnung und einer Auslassöffnung im Bereich eines zweiten Schneckenrohrendes, und eine sich in der Längsrichtung erstreckende und in dem Schneckenrohr angeordnete Förderschnecke, die drehbar gelagert ist und mittels eines Schneckenantriebs in Rotation versetzt werden kann, welcher sich dadurch auszeichnet, dass die Einlassöffnung als radiale Öffnung in dem Schneckenrohr ausgebildet ist und sich über einen Einlassöffnungsabschnitt in Längsrichtung des Schneckenrohres erstreckt und dass die Förderschnecke im Bereich des Einlassöffnungsabschnitts eine erste und eine zweite, von der ersten verschiedene Schneckensteigung aufweist. Dieser Förderer kann insbesondere ausgebildet sein nach den Merkmalen der zuvor beschriebenen Ausführungsformen des erfindungsgemäßen Schneckenförderers. Die Ausführungsform des Schneckenförderer definiert eine spezifische Lösung für eine Problematik, die im Bereich der Zufuhr von Feststoffen über einen offenen Schneckentrog, der eine radiale Einlassöffnung ausbildet, auftreten kann. Im Falle einer solchen radialen Zuführung über einen definierten axialen Bereich kann bei bestimmten Feststoffen oftmals eine unregelmäßige Aufnahme der Feststoffe aus dem Schneckentrog bzw. aus der Einlassöffnung beobachtet werden, die eine zunächst erfolgende Aufnahme am endseitigen Einlassbereich des Schneckenförderer stattfinden lässt und erst nach endseitiger Leerung des Schneckentrogs eine Aufnahme von Material aus den Bereichen des Schneckentrogs ermöglicht, die sich von dem endseitigen Ende des Schneckentrogs in Richtung des zweiten Endes des Schneckenförderers erstrecken. Diese so beobachtete Wanderung des Füllpunktes von hinten nach vorne kann bei vorgeschalteten Beschickungsaggregaten, wie beispielsweise einem Schubboden, zu Schwierigkeiten führen und kann insbesondere Steuerungs- und Regelungsvorgänge, die eine qualitative oder quantitative Fördermengensteuerung oder -regelung zum Ziel haben, erheblich verkomplizieren. Zudem besteht aufgrund dieser Problematik die Gefahr, dass der Schneckentrog abschnittsweise überfüllt wird und überläuft oder nicht ausreichend befüllt ist, was zu einer Leerförderung führen könnte. Eine solche Leerförderung wiederum ruft konkret eine unzureichende Abdichtung hervor, was erfindungsgemäß vermieden werden soll.

Durch die erfindungsgemäß bereitgestellte Ausführung des Schneckenförderers im Bereich der Einlassöffnung wird diese ungleichmäßige Aufnahme der Feststoffe aus dem Schneckentrog durch eine spezifische Ausgestaltung der Schneckenflügel verhindert. In Anpassung an die unterschiedlichen Füll- und Aufnahmeverhältnisse wird erfindungsgemäß dabei die Steigung angepasst, insbesondere kann dabei vorgesehen sein, dass Bereiche, die nicht eine effiziente Aufnahme aus dem Schneckentrog erzielen mit einer höheren Schneckensteigerung versehen werden als Bereiche, die bevorzugt aus dem Schneckentrog befüllt werden, die eine entsprechend kleinere Steigerung aufweisen.

Dabei kann insbesondere vorgesehen sein, dass sich die Steigung der Schnecke im Bereich des Einlassöffnungsabschnitts kontinuierlich ändert. Durch eine solche kontinuierliche Änderung kann eine vor Blockaden sichere Förderung erreicht werden, die in wirksamer Weise eine sich kontinuierlich ändernde Aufnahmeeffizienz entlang des Einfülltrichters gegensteuern kann.

Noch weiter ist bevorzugt vorgesehen, dass die Steigung der Schnecke in dem zum ersten Schneckenrohrende weisenden Bereich des Einlassöffnungsabschnitts kleiner ist als in dem zum zweiten Schneckenrohrende weisenden Bereich des Einlassöffnungsabschnitts. Mit dieser Ausführungsform wird die häufig beobachtete Problematik, dass benachbart zum ersten Ende eine verstärkte Aufnahme aus dem Schneckentrog erfolgt, wirksam begegnet.

Ein weiterer Aspekt der Erfindung ist eine Biogasanlage, umfassend einen gasdicht verschließbaren Fermenter mit einer Feststoffzufuhröffnung einer Gasableitungsöffnung, welcher erfindungsgemäß gekennzeichnet ist durch einen Schneckenförderer nach einem der vorhergehenden Ansprüche, dessen Auslassöffnung unmittelbar mit der Feststoffzufuhröffnung oder einer unter Fermenterinnendruck stehenden Zuleitung zur Feststoffzufuhröffnung des Fermenters verbunden ist. Mit der so erfindungsgemäß bereitgestellten Biogasanlage wird eine zuverlässige kontinuierliche oder quasikontinuierlich Förderung von organischen Feststoffen in einen Fermenter ermöglicht und hierbei eine sichere Abdichtung gegen unerwünschten Austritt von Feststoffen, Flüssigkeiten oder Gasen durch den Schneckenförderer erzielt, wenn der Schneckenförderer in Betrieb ist oder außer Betrieb ist.

Der erfindungsgemäße Schneckenförderer arbeitet vorzugsweise nach einem Verfahren zum Zuführen von organischen Feststoffen in einen unter Innendruck stehenden Fermenter, mit den Schritten: Fördern der organischen Stoffe mittels eines Schneckenförderers von einer Einlassöffnung zu einer gasdicht mit dem Fermenter unmittelbar verbundenen Auslassöffnung, Verdichten der organischen Stoffe in dem Schneckenförderer zur Verhinderung oder Verminderung des Gasdurchgangs durch den Schneckenförderer, wobei das Verdichten der organischen Stoffe vorzugsweise durch eine Verringerung des Durchtrittsquerschnitts des Schneckenförderers in Förderrichtung erfolgt und weiter vorzugsweise der Durchtrittsquerschnitt in Förderrichtung vor der Verringerung erweitert wird, optionales Separieren von Flüssigkeit aus den organischen Stoffen durch mehrere radiale Öffnungen in dem Schneckenförderer vor dem Verdichten, optionales Einspülen einer Flüssigkeit nach dem Verdichten.

Eine bevorzugte Ausführungsform der Erfindung wird anhand der beiliegenden Figuren erläutert. Es zeigen:
Figur 1 eine vertikal längsgeschnittene Seitenansicht eines erfindungsgemäßen Schneckenförderers,
Figur 2 eine horizontal teil-längsgeschnittene Draufsicht auf den Schneckenförderer gemäß Figur 1,
Figur 3 eine teilgeschnittene, perspektivische Ansicht des Schneckenförderers gemäß den Figuren 1 und 2.
Figur 4 eine Detailansicht gemäß des in Figur 1 markierten Details Z,
Figur 5a eine quergeschnittene Ansicht entlang der Linie A-A in Figur 1 mit montierten Schneckenflügeln, und
Figur 5b eine quergeschnittene Ansicht entlang der Linie A-A in Figur 1 mit demontierten Schneckenflügeln.

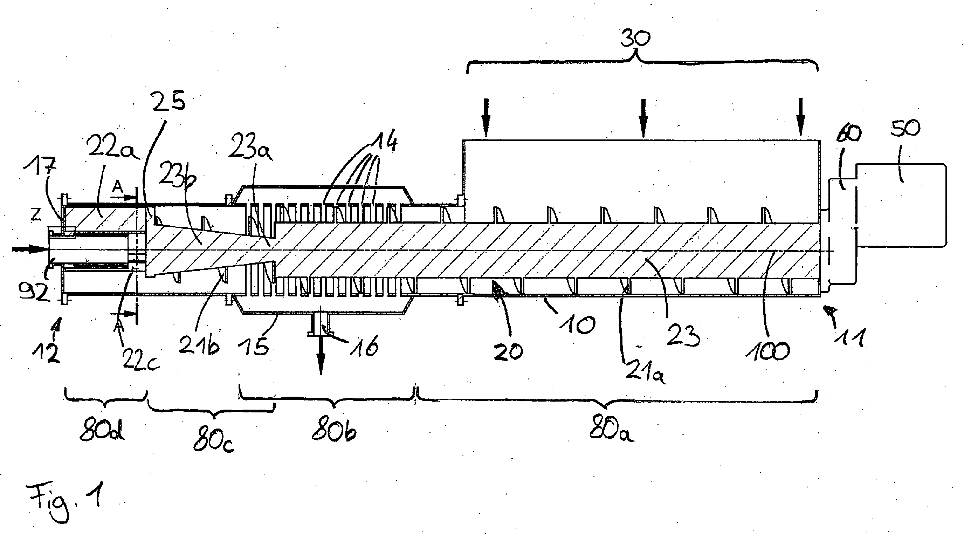

In den Figuren 1, 2 und 3 ist ein Schneckenförderer 1 abgebildet, der grundsätzlich ein Schneckenrohr 10 umfasst, in dem eine Förderschnecke 20 drehbar um eine Längsachse 100 gelagert angeordnet ist. Die Förderschnecke umfasst einen Förderschneckenkern 23 und Schneckenflügel 21a, b, 22a-c.

Eine Einlassöffnung 30 erstreckt sich ausgehend von einem ersten Ende 11 des Schneckenrohrs 10 in axialer Richtung in Richtung eines zweiten Endes 12 des Schneckenrohrs 10 über etwa die Hälfte der Gesamtlänge des Schneckenrohres 10. Die Einlassöffnung 30 ist als Schneckentrog 13 ausgebildet und ermöglicht eine radiale Zufuhr von organischen Feststoffen in das Schneckenrohr 10.

Im Bereich des zweiten Endes 12 ist eine als Rohrflansch ausgebildete Auslassöffnung 40 angeordnet, die sich ebenfalls radial erstreckt und deren Austrittsrichtung um 90 Grad gegenüber der Längsachse 100 zu der Eintrittsrichtung der organischen Feststoffe in die Einlassöffnung 30 verdreht ist.

Am ersten Ende 11 des Schneckenrohres 10 ist ein elektrischer Antriebsmotor 50 über ein Zwischengetriebe 60 an das Gehäuserohr angeflanscht. Der Antriebsmotor 50 setzt über das Zwischengetriebe 60 die Förderschnecke 20 in Rotation um die Längsachse 100.

Die Förderschnecke 20 weist einen ersten Förderabschnitt 80a auf, der sich über den gesamten Bereich der Einlassöffnung erstreckt und weiterhin einen daran anschließenden Bereich umfasst, in dem die Förderschnecke 20 von einem geschlossenen Rohrabschnitt des Förderrohrs umgeben ist. An diesem ersten Schneckenförderabschnitt 80a weist die Förderschnecke 20 einen konstanten Durchmesser des Förderschneckenkerns 23 und einen konstanten Außendurchmesser der darauf angeordneten Schneckenflügel 21a auf. Die Schneckenflügel 21a sind als ein eingängiges Gewinde, welches sich in Längsrichtung um den Förderschneckenkern windet, ausgeführt.

Es ist zu verstehen, dass die Steigung in diesem ersten Förderschneckenabschnitt 80a über die gesamte Länge für die Schneckenflügel 21a konstant sein kann. Ebenso kann aber in einem benachbart zum Motor 50 liegenden Abschnitt des ersten Schneckenförderabschnitts 80a eine geringere Gewindesteigung vorgesehen sein als in einem hierzu in Förderrichtung weiter fortgeschritten liegenden Abschnitt des Schneckenförderabschnitts 80a, um eine axial gleichmäßige Entleerung des Schneckentrogs 13 zu erzielen.

An den ersten Schneckenförderabschnitt 80a schließt sich in Förderrichtung ein zweiter Schneckenförderabschnitt 80b an. In diesem zweiten Abschnitt 80b ist das Schneckenrohr durch mehrere Öffnungen 14 unterbrochen und von einem Gehäuse 15 umgeben. In diesem Separationsabschnitt 80b tritt Flüssigkeit durch die Öffnungen 14 aus dem Schneckenrohr aus, wodurch eine Entwässerung der organischen Feststoffe erzielt wird. Die ausgetretene Flüssigkeit wird in dem Gehäuse 15 aufgefangen und durch einen in Schwerkraftrichtung nach unten weisenden Stutzen 16 abgeführt.

An diesen zweiten Schneckenförderabschnitt 80b schließt sich in Förderrichtung ein dritter Schneckenförderabschnitt 80c an, der sich funktionell mit dem Separationsabschnitt 80b teilweise überschneiden kann. Während die Förderschnecke im zweiten Abschnitt 80b ebenso ausgeführt ist wie im ersten Abschnitt 80a, reduziert sich der Durchmesser des Förderschneckenkerns 23 im dritten Abschnitt 80c durch einen Absatz stufig auf einen deutlich reduzierten Kerndurchmesser in einem Bereich 23a. Diese Reduktion erfolgt im Eingangsbereich des dritten Abschnitts 80c und bewirkt eine Vergrößerung des Durchtrittsquerschnitts. Ausgehend von dieser Reduktion erweitert sich der Durchmesser des Förderschneckenkerns wieder auf den ursprünglichen Durchmesser des Förderschneckenkerns, der auch im ersten und zweiten Abschnitt 80a, b konstant vorliegt. Diese Erweiterung erfolgt durch eine konische Ausgestaltung in einem Bereich 23b über praktisch die gesamte Länge des dritten Abschnitts 80c. Die Vergrößerung bewirkt eine Verringerung des Durchtrittsquerschnitts.

In diesem dritten Abschnitt 80c, der als Stopfabschnitt funktioniert, können Schneckenflügel 21 b mit einer in Förderrichtung abnehmenden Steigung ausgeführt sein, um den Verdichtungseffekt zu unterstützen. Die Schneckenflügel 21 b können im Abschnitt 80c einen kleineren Außendurchmesser auf weisen als im Abschnitt 80b und können mit konstantem Außendurchmesser ausgeführt. Sein. Dies hat zur Folge, dass aufgrund des sich vergrößernden Kerndurchmessers im Stopfabschnitt 80c die Schneckenflügel eine in Förderrichtung abnehmende radiale Tiefe aufweisen. Das Schneckenrohr ist im Anfangsbereich des Stopfabschnitts 80c mit kleinen Öffnungen ausgeführt sodass sich Stopfabschnitt 80c und Separationsabschnitt 80b funktionell teilweise überschneiden. In einem hieran in Förderrichtung anschließenden Mittel- und Endbereich des Stopfabschnitts 80c ist das Schneckenrohr, wiederum geschlossen ausgeführt wie in Abschnitt 80a.

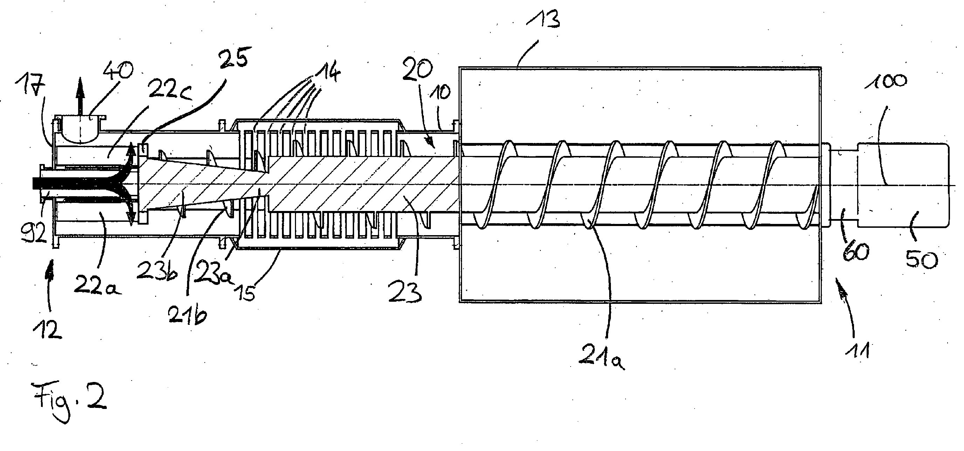

Innerhalb des Stopfabschnitts 80c bildet sich an dessen in Förderrichtung hinten liegendem (in Figur 1 und 2 linken) Ende ein verdichteter Stopfen aus organischen Feststoffen, der eine zuverlässige Abdichtung gegen unerwünschten Durchtritt von Feststoffen, Flüssigkeiten oder Gasen während des Betriebs und im Stillstand des Schneckenförderers bewirkt.

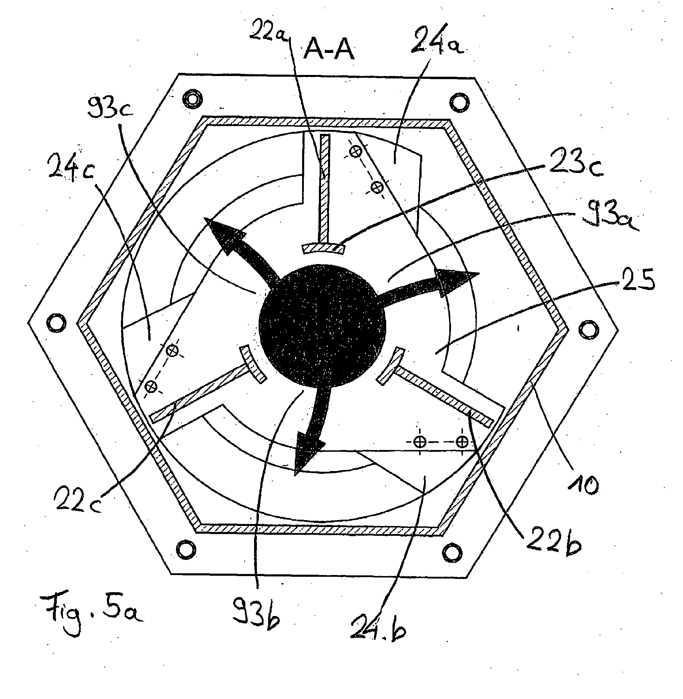

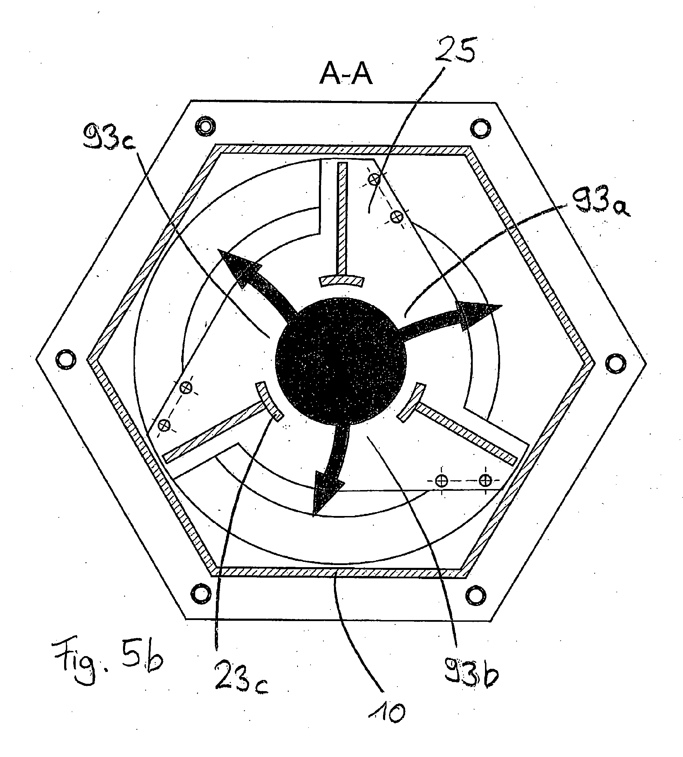

An den Stopfabschnitt 80c schließt sich ein vierter Schneckenförderabschnitt 80d in Förderrichtung an, der als Auswurfabschnitt funktioniert. Im Auswurfabschnitt 80d sind, wie insbesondere aus Figur 5a, b zu erkennen, drei Schneckenflügel 22a-c montiert, die sich in axialer Richtung erstrecken und eine größere Steigung als die Schneckenflügel 21a, b aufweisen. Diese drei Schneckenflügel 22a-c sind an dem Im Abschnitt 80d als Hohlwelle 23c ausgebildeten Kern der Förderschnecke befestigt.

Zwischen dem Stopfabschnitt 80c und dem Auswurfabschnitt 80d ist weiterhin ein Trägerflansch 25 an der Förderschnecke 20 drehmomentfest befestigt. An dem Trägerflansch sind jeweils zwei Bohrungen um 120° über den Umfang verteilt ausgebildet, die dazu dienen, daran wahlweise drei Schneckenflügel 24a-c zu montieren und auszutauschen, beispielsweise um den in den Figur 5a,b ersichtlichen Durchtrittsquerschnitt mittels der drei Schneckenflügel 22a-c an die Konsistenz der geförderten Feststoffe anzupassen.

Die Schneckenflügel 22a-c und 24a-c bewirken einen wirksamen Auswurf der geförderten organischen Feststoffe durch die Auslassöffnung 40 nach radial auswärts. Die Schneckenflügel 24a-c können dabei zusätzlich eine Zerkleinerung der geförderten Feststoffe bewirken und folglich als rotierende Schneidmesser arbeiten.

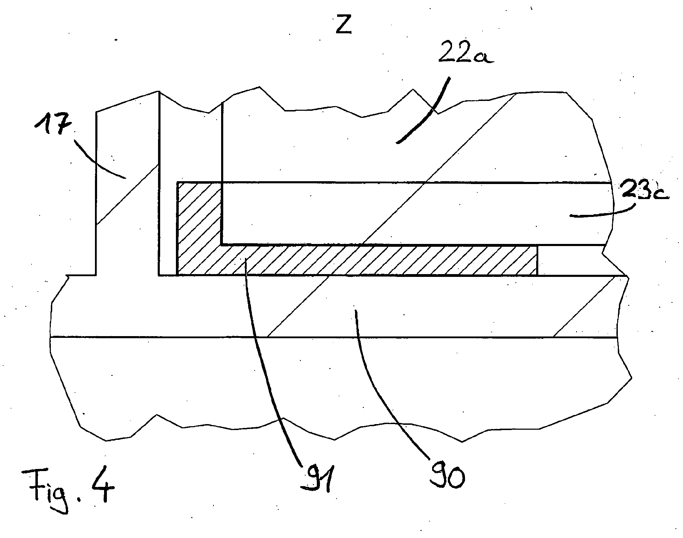

Im Bereich des Auswurfsabschnitts 80d ist die Förderschnecke 20 als Hohlwelle 23c ausgeführt und umschließt einen Anschlussstutzen 90, der drehmomentfest in eine stirnseitige Wandung 17 am Ende 12 des Schneckenrohrs montiert ist. Wie insbesondere aus Figur 4 ersichtlich, ist die Förderschnecke 20 mittels einer Gleitlagerbuchse 91 drehbar auf dem Stutzen 90 in diesem Bereich des zweiten Endes 12 gelagert.

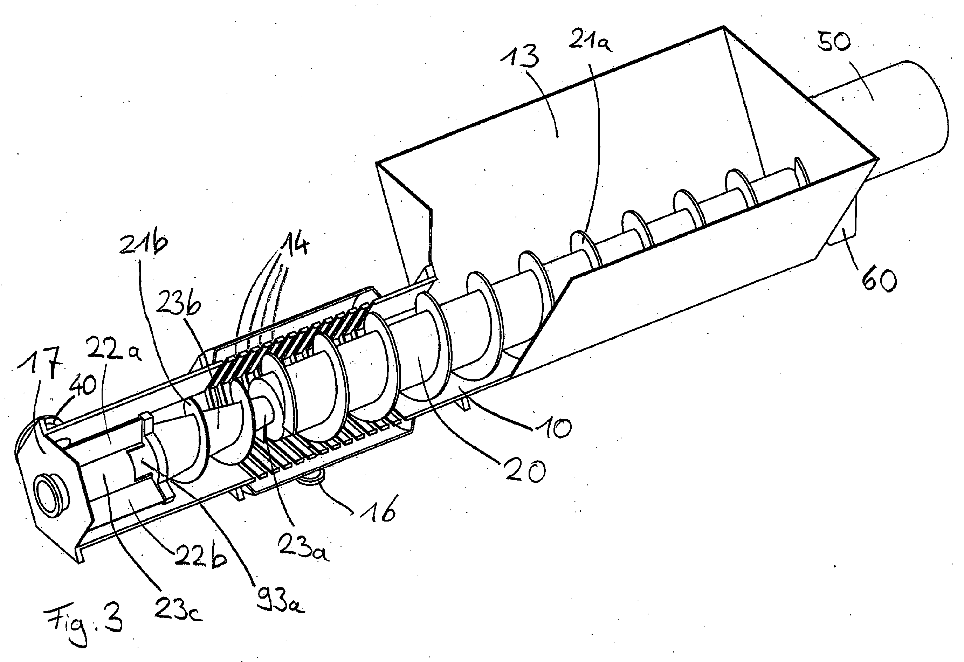

Der Stutzen 90 weist eine Einlassöffnung 92 auf, durch die eine Spülflüssigkeit in axialer Richtung eingespült werden kann. Diese Spülflüssigkeit wird, wie durch den sich verzweigenden Pfeil in Figur 2 verdeutlicht, durch den Stutzen 90 hindurch geführt und in radialer Richtung in den Durchtrittsquerschnitt im Bereich des Auswurfsabschnitts 80d ausgeführt. Wie aus Figur 3 ersichtlich, sind hierzu drei radiale Auslassöffnungen 93a-c an der als Hohlwelle ausgeführten Förderschnecke vorgesehen, die jeweils zwischen den Schneckenflügeln 22a-c einen Flüssigkeitsaustritt bewirken. Durch diese so eingeführte Spülflüssigkeit wird das zuvor verdichtete organische Feststoffmaterial verflüssigt und in seiner Viskosität verringert, um einen wirksamen Auswurf durch die Auslassöffnung 40 zu erreichen.

Aus Figur 3 ist weiterhin ersichtlich, dass das Schneckenrohr 10 im Querschnitt sechseckig ausgeführt ist. Die Schneckenflügel 21a, 22a-c und 24a-c weisen hierbei einen Außendurchmesser auf, der mit geringfügigem Spiel einem in dieses so definierte Sechseck eingeschriebenem Kreis entspricht. Der Durchtrittsquerschnitt durch den Schneckenförderer wird folglich durch eine äußere, sechseckige Begrenzung durch das Schneckenrohr und eine innere, kreisrunde Begrenzung durch den Kern der Förderschnecke definiert und weiter durch den Querschnitt der Schneckenflügel 21 a, b, 24a-c und 22a-c geringfügig reduziert.

## Patentansprüche

1. Schneckenförderer zum Einbringen organischer Stoffe in einen unter Gasinnendruck stehenden Biogasfermenter, der Schneckenförderer umfassend:
- Ein sich in einer Längsrichtung erstreckendes Schneckenrohr (10) mit einer Einlassöffnung (30) für organische Stoffe im Bereich eines ersten Schneckenrohrendes (11) und einer Auslassöffnung (40) für die organischen Stoffe im Bereich eines zweiten Schneckenrohrendes 12),
- Eine sich in der Längsrichtung erstreckende und in dem Schneckenrohr angeordnete Förderschnecke (20), die drehbar gelagert ist und mittels eines Schneckenantriebs (50, 60) in Rotation versetzt werden kann,
- Eine Fermenteranschluss (40) zum direkten Verbinden der Auslassöffnung mit dem unter Gasdruck stehenden Biogasfermenter,
- Einen Stopfabschnitt (80c) in einem Bereich zwischen Einlass- und Auslassöffnung zum Verdichten der organischen Stoffe zur Verringerung eines Gasdurchtritts durch den Schneckenförderer,
**dadurch gekennzeichnet dass** der Stopfabschnitt einen Erweiterungsbereich (23a) aufweist, in dem sich der Durchtrittsquerschnitt zwischen der Förderschnecke und dem Schneckenrohr in Förderrichtung erweitert und einen in Förderrichtung hinter dem Erweiterungsbereich angeordneten Verengungsbereich (23b) aufweist, in dem sich der Durchtrittsquerschnitt zwischen der Förderschnecke und dem Schneckenrohr in Förderrichtung reduziert.

2. Schneckenförderer nach Anspruch 1,
**dadurch gekennzeichnet dass** die Förderschnecke
- in einem Bereich unmittelbar in Förderrichtung zu Beginn des Stopfabschnitts einen ersten Durchtrittsquerschnitt zwischen der Förderschnecke und dem Schneckenrohr aufweist,
- hinter dem ersten Durchtrittsquerschnitt im Anfangsbereich des Stopfabschnitts einen zweiten, gegenüber dem ersten Durchtrittsquerschnitt vergrößerten Durchtrittsquerschnitt aufweist und
- am Ende des Stopfabschnitts einen dritten, gegenüber dem zweiten verringerten Durchtrittsquerschnitt aufweist.

3. Schneckenförderer nach Anspruch 1 oder 2,
**dadurch gekennzeichnet dass** sich der Durchtrittsquerschnitt im Stopfabschnitt in Förderrichtung im Anfangsbereich stufig erweitert und/oder über den Großteil des Stopfabschnitts kontinuierlich verringert.

4. Schneckenförderer einem der vorhergehenden Ansprüche,
**dadurch gekennzeichnet dass** sich der Durchtrittsquerschnitt vor dem Stopfabschnitt ausgehend von einem ersten Durchtrittsquerschnitt, der eine durch ein erstes Kugeldurchgangsmaß gekennzeichnete Querschnittsgeometrie aufweist, auf einen zweiten Durchtrittsquerschnitt mit einer Querschnittsgeometrie, die durch zweites Kugeldurchgangsmaß gekennzeichnet ist, das größer als das erste Kugeldurchgangsmaß ist, ändert und weiter auf einen dritten Durchtrittsquerschnitt mit einer Querschnittsgeometrie, die durch ein drittes Kugeldurchgangsmaß gekennzeichnet ist, das kleiner als das zweite Kugeldurchgangsmaß ist, ändert, wobei das erste und das dritte Kugeldurchgangsmaß vorzugsweise übereinstimmend sind.

5. Schneckenförderer nach einem der vorhergehenden Ansprüche, der Schneckenförderer umfassend:
- Ein sich in einer Längsrichtung erstreckendes Schneckenrohr mit einer Einlassöffnung für organische Stoffe im Bereich eines ersten Schneckenrohrendes und einer Auslassöffnung für die organischen Stoffe im Bereich eines zweiten Schneckenrohrendes,
- Eine sich in der Längsrichtung erstreckende und in dem Schneckenrohr angeordnete Förderschnecke, die drehbar gelagert ist und mittels eines Schneckenantriebs in Rotation versetzt werden kann,
- Eine Fermenteranschluss zum direkten Verbinden der Auslassöffnung mit dem unter Gasdruck stehenden Biogasfermenter,
- Einen Stopfabschnitt zum Verdichten der organischen Stoffe zur Verringerung eines Gasdurchtritts durch den Schneckenförderer,
**gekennzeichnet durch** einen in Förderrichtung vor dem Stopfabschnitt angeordneten Separationsabschnitt, umfassend eine Mehrzahl von Öffnungen im Schneckenrohr und eine Flüssigkeitsauffangvorrichtung außerhalb des Schneckenrohres.

6. Schneckenförderer nach einem der vorhergehenden Ansprüche, der Schneckenförderer umfassend:
- Ein sich in einer Längsrichtung erstreckendes Schneckenrohr mit einer Einlassöffnung für organische Stoffe im Bereich eines ersten Schneckenrohrendes und einer Auslassöffnung für die organischen Stoffe im Bereich eines zweiten Schneckenrohrendes,
- Eine sich in der Längsrichtung erstreckende und in dem Schneckenrohr angeordnete Förderschnecke, die drehbar gelagert ist und mittels eines Schneckenantriebs in Rotation versetzt werden kann,
- Eine Fermenteranschluss zum direkten Verbinden der Auslassöffnung mit dem unter Gasdruck stehenden Biogasfermenter,
- Einen Stopfabschnitt zum Verdichten der organischen Stoffe zur Verringerung eines Gasdurchtritts durch den Schneckenförderer,
**gekennzeichnet durch** eine Spülflüssigkeitszufuhröffnung zum Zuführen einer Spülflüssigkeit in den Durchtrittsquerschnitt zwischen Schneckenrohr und Förderschnecke im Bereich des zweiten Schneckenrohrendes in Förderrichtung hinter dem Stopfabschnitt.

7. Schneckenförderer nach Anspruch 6,
**dadurch gekennzeichnet dass** eine Spüleinlassöffnung an der Stirnseite des Schneckenrohres angeordnet ist und in eine axial in Längsrichtung des Schneckenrohres verlaufende Spülleitung mündet, die sich ausgehend vom zweiten Ende des Schneckenrohres zu mindestens einer radialen Spülflüssigkeitszufuhröffnung erstreckt, die in den Durchtrittsquerschnitt zwischen Förderschnecke und Schneckenrohr mündet.

8. Schneckenförderer nach Anspruch 6 oder 7,
**dadurch gekennzeichnet dass** die Förderschnecke im Bereich des zweiten Endes als Hohlwelle ausgebildet ist zur Leitung der Spülflüssigkeit im Innenraum der Förderschnecke.

9. Schneckenförderer nach einem der Ansprüche 7 bis 9,
**dadurch gekennzeichnet dass** die Förderschnecke im Bereich des zweiten Endes auf einem Stutzen drehbar gelagert ist, der drehmomentfest am Schneckenrohr befestigt ist und sich axial in einen Hohlraum der Förderschnecke erstreckt.

10. Schneckenförderer nach einem der vorhergehenden Ansprüche, der Schneckenförderer umfassend:
- Ein sich in einer Längsrichtung erstreckendes Schneckenrohr mit einer Einlassöffnung für organische Stoffe im Bereich eines ersten Schneckenrohrendes und einer Auslassöffnung für die organischen Stoffe im Bereich eines zweiten Schneckenrohrendes,
- Eine sich in der Längsrichtung erstreckende und in dem Schneckenrohr angeordnete Förderschnecke, die drehbar gelagert ist und mittels eines Schneckenantriebs in Rotation versetzt werden kann,
- Eine Fermenteranschluss zum direkten Verbinden der Auslassöffnung mit dem unter Gasdruck stehenden Biogasfermenter,
- Einen Stopfabschnitt zum Verdichten der organischen Stoffe zur Verringerung eines Gasdurchtritts durch den Schneckenförderer,
**gekennzeichnet durch** zumindest einen Schneckenflügel im Bereich des zweiten Schneckenrohrendes, der lösbar an der Förderschnecke befestigt ist.

11. Schneckenförderer nach Anspruch 10,
**gekennzeichnet durch** einen ersten auf dem Schneckenförderer montierbaren Schneckenflügel und einen zweiten, alternativ zum ersten montierbaren Schneckenflügel, der einen Querschnitt aufweist, der gegenüber dem ersten Schneckenflügel eine größere Querschnittsfläche des Durchtrittsquerschnitts zwischen Förderschnecke und Schneckenrohr ausfüllt.

12. Schneckenförderer nach einem der vorhergehenden Ansprüche, der Schneckenförderer umfassend:
- Ein sich in einer Längsrichtung erstreckendes Schneckenrohr mit einer Einlassöffnung für organische Stoffe im Bereich eines ersten Schneckenrohrendes und einer Auslassöffnung für die organischen Stoffe im Bereich eines zweiten Schneckenrohrendes,
- Eine sich in der Längsrichtung erstreckende und in dem Schneckenrohr angeordnete Förderschnecke, die drehbar gelagert ist und mittels eines Schneckenantriebs in Rotation versetzt werden kann,
- Eine Fermenteranschluss zum direkten Verbinden der Auslassöffnung mit dem unter Gasdruck stehenden Biogasfermenter,
- Einen Stopfabschnitt zum Verdichten der organischen Stoffe zur Verringerung eines Gasdurchtritts durch den Schneckenförderer,
**dadurch gekennzeichnet, dass** die Innenwandung des Schneckenrohrs eine nicht-kreisrunde innere Querschnittsfläche umschließt.

13. Schneckenförderer nach Anspruch 12,
**dadurch gekennzeichnet, dass** die Innenwandung des Schneckenrohrs eine eckige innere Querschnittsfläche umschließt.

14. Schneckenförderer, nach einem der vorhergehenden Ansprüche, der Schneckenförderer umfassend:
- Ein sich in einer Längsrichtung erstreckendes Schneckenrohr mit einer Einlassöffnung und einer Auslassöffnung im Bereich eines zweiten Schneckenrohrendes,
- Eine sich in der Längsrichtung erstreckende und in dem Schneckenrohr angeordnete Förderschnecke, die drehbar gelagert ist und mittels eines Schneckenantriebs in Rotation versetzt werden kann,
**dadurch gekennzeichnet, dass** die Einlassöffnung als radiale Öffnung in dem Schneckenrohr ausgebildet ist und sich über einen Einlassöffnungsabschnitt in Längsrichtung des Schneckenrohres erstreckt und dass die Förderschnecke im Bereich des Einlassöffnungsabschnitts eine erste und eine zweite, von der ersten verschiedene Schneckensteigung aufweist.

15. Schneckenförderer nach Anspruch 14,
**dadurch gekennzeichnet, dass** sich die Steigung der Schnecke im Bereich des Einlassöffnungsabschnitts kontinuierlich ändert.

16. Schneckenförderer nach Anspruch 14 oder 15,
**dadurch gekennzeichnet, dass** die Steigung der Schnecke in dem zum ersten Schneckenrohrende weisenden Bereich des Einlassöffnungsabschnitts kleiner ist als in dem zum zweiten Schneckenrohrende weisenden Bereich des Einlassöffnungsabschnitts.

17. Biogasanlage, umfassend einen gasdicht verschließbaren Fermenter mit einer Feststoffzufuhröffnung einer Gasableitungsöffnung,
**gekennzeichnet durch** einen Schneckenförderer nach einem der vorhergehenden Ansprüche, dessen Auslassöffnung unmittelbar mit der Feststoffzufuhröffnung oder einer unter Fermenterinnendruck stehenden Zuleitung zur Feststoffzufuhröffnung des Fermenters verbunden ist.

18. Verfahren zum Zuführen von organischen Feststoffen in einen unter Innendruck stehenden Fermenter, mit den Schritten:
- Fördern der organischen Stoffe mittels eines Schneckenförderers von einer Einlassöffnung zu einer gasdicht mit dem Fermenter unmittelbar verbundenen Auslassöffnung,
- Verdichten der organischen Stoffe in dem Schneckenförderer zur Verhinderung oder Verminderung des Gasdurchgangs durch den Schneckenförderer,
- Wobei das Verdichten der organischen Stoffe durch eine Verringerung des Durchtrittsquerschnitts des Schneckenförderers in Förderrichtung erfolgt und weiter der Durchtrittsquerschnitt in Förderrichtung vor der Verringerung er- ' weitert wird,
- Optionales Separieren von Flüssigkeit aus den organischen Stoffen durch mehrere radiale Öffnungen in dem Schneckenförderer vor dem Verdichten,
- Optionales Einspülen einer Flüssigkeit nach dem Verdichten.

## Claims

1. Screw conveyor for introducing organic matter into a biogas digester which is under internal gas pressure, said screw conveyor comprising:
a screw pipe (10) extending in a longitudinal direction and having an inlet opening (30) for organic matter in the region of a first screw pipe end (11) and an outlet opening (40) for the organic matter in the region of a second screw pipe end (12),
a conveying screw (20) which extends in the longitudinal direction, is arranged in the screw pipe, is rotatably mounted and can be made to rotate by means of a screw drive (50, 60),
a digester connector (40) for connecting the outlet opening directly to the biogas digester which is under gas pressure,
a plug section (80c) in a region between the inlet opening and the outlet opening for compacting the organic matter in order to reduce any passage of gas through the screw conveyor,
**characterised in that** the plug section has an expansion region (23a) in which the passage cross-section between the conveying screw and the screw pipe widens in the conveying direction, and a constriction region (23b) which is located, in the conveying direction, downstream of the expansion region and in which the passage cross-section between the conveying screw and the screw pipe is reduced in the conveying direction.

2. Screw conveyor as claimed in claim 1,
**characterised in that** the conveying screw
has a first passage cross-section between the conveying screw and the screw pipe in a region immediately at the beginning of the plug section in the conveying direction,
has a second passage cross-section, which is enlarged relative to the first passage cross-section, downstream of the first passage cross-section in the initial region of the plug section, and
has a third passage cross-section, reduced in relation to the second passage crosssection, at the end of the plug section.

3. Screw conveyor as claimed in Claim 1 or 2,
**characterised in that** the passage cross-section in the plug section is expanded in steps in the conveying direction in the initial region and/or is continuously reduced over the majority of the plug section.

4. Screw conveyor as claimed in any one of the preceding claims,
**characterised in that** the passage cross-section changes upstream of the plug section, starting from a first passage cross-section having a cross-sectional geometry **characterised by** a first ball passage dimension, to a second passage cross-section having a cross-sectional geometry **characterised by** a second ball passage dimension which is larger than the first ball passage dimension, and changes further to a third passage cross-section having a cross-sectional geometry **characterised by** a third ball passage dimension which is smaller than the second ball passage dimension, wherein the first and the third ball passage dimensions are preferably congruent.

5. Screw conveyor as claimed in any one of the preceding claims, said screw conveyor comprising:
a screw pipe extending in a longitudinal direction and having an inlet opening for organic matter in the region of a first screw pipe end and an outlet opening for the organic matter in the region of a second screw pipe end,
a conveying screw which extends in the longitudinal direction, is arranged in the screw pipe, is rotatably mounted and can be made to rotate by means of a screw drive,
a digester connector for connecting the outlet opening directly to the biogas digester which is under gas pressure,
a plug section for compacting the organic matter in order to reduce any passage of gas through the screw conveyor,
**characterised by** a separation section disposed upstream of the plug section in the conveying direction, comprising a plurality of openings in the screw pipe and a fluid collection apparatus outside the screw pipe.

6. Screw conveyor as claimed in any one of the preceding claims, said screw conveyor comprising:
a screw pipe extending in a longitudinal direction and having an inlet opening for organic matter in the region of a first screw pipe end and an outlet opening for the organic matter in the region of a second screw pipe end,
a conveying screw which extends in the longitudinal direction, is arranged in the screw pipe, is rotatably mounted and can be made to rotate by means of a screw drive,
a digester connector for connecting the outlet opening directly to the biogas digester which is under gas pressure,
a plug section for compacting the organic matter in order to reduce any passage of gas through the screw conveyor,
**characterised by** a flushing fluid feed opening for feeding a flushing fluid into the passage cross-section between the screw pipe and the conveying screw in the region of the second screw pipe end downstream of the plug section in the conveying direction.

7. Screw conveyor as claimed in claim 6,
**characterised in that** a flushing inlet opening is arranged at the end face of the screw pipe and opens into a flushing line extending axially in the longitudinal direction of the screw pipe, said flushing line extending from the second end of the screw pipe to at least one radial flushing fluid feed opening which opens into the passage cross-section between the conveying screw and the screw pipe.

8. Screw conveyor as claimed in Claim 6 or 7,
**characterised in that** the conveying screw is formed in the region of the second end as a hollow shaft for conducting the flushing fluid in the interior of the conveying screw.

9. Screw conveyor as claimed in any one of claims 7 to 9,
**characterised in that** the conveying screw is rotatably mounted in the region of the second end on a pipe connection which is fixed in a torque-resistant manner to the screw pipe and which extends axially into a hollow space in the conveying screw.

10. Screw conveyor as claimed in any one of the preceding claims, said screw conveyor comprising:
a screw pipe extending in a longitudinal direction and having an inlet opening for organic matter in the region of a first screw pipe end and an outlet opening for the organic matter in the region of a second screw pipe end,
a conveying screw which extends in the longitudinal direction, is arranged in the screw pipe, is rotatably mounted and can be made to rotate by means of a screw drive,
a digester connector for connecting the outlet opening directly to the biogas digester which is under gas pressure,
a plug section for compacting the organic matter in order to reduce any passage of gas through the screw conveyor,
**characterised by** at least one flight which is disposed in the region of the second screw pipe end and which is detachably fixed to the conveying screw.

11. Screw conveyor as claimed in claim 10,
**characterised by** a first flight which can be mounted on the screw conveyor and by a second flight which can be mounted alternatively to the first and which has a cross-section which fills out a larger cross-sectional area, relative to the first flight, of the passage cross-section between the conveying screw and the screw pipe.

12. Screw conveyor as claimed in any one of the preceding claims, said screw conveyor comprising:
a screw pipe extending in a longitudinal direction and having an inlet opening for organic matter in the region of a first screw pipe end and an outlet opening for the organic matter in the region of a second screw pipe end,
a conveying screw which extends in the longitudinal direction, is arranged in the screw pipe, is rotatably mounted and can be made to rotate by means of a screw drive,
a digester connector for connecting the outlet opening directly to the biogas digester which is under gas pressure,
a plug section for compacting the organic matter in order to reduce any passage of gas through the screw conveyor,
**characterised in that** the inner wall of the screw pipe encloses a non-circular inner crosssectional area.

13. Screw conveyor as claimed in claim 12,
**characterised in that** the inner wall of the screw pipe encloses a polygonal inner cross-sectional area.

14. Screw conveyor as claimed in any one of the preceding claims, said screw conveyor comprising:
a screw pipe extending in a longitudinal direction and having an inlet opening and
an outlet opening in the region of a second screw pipe end,
a conveying screw which extends in the longitudinal direction, is arranged in the screw pipe, is rotatably mounted and can be made to rotate by means of a screw drive,
**characterised in that** the inlet opening is designed as a radial opening in the screw pipe and extends across an inlet opening section in the longitudinal direction of the screw pipe, and that in the region of the inlet opening section the conveying screw has a first screw pitch and a second screw pitch differing from the first screw pitch.

15. Screw conveyor as claimed in claim 14,
**characterised in that** that the screw pitch continuously changes in the region of the inlet opening section.

16. Screw conveyor as claimed in Claim 14 or 15,
**characterised in that** the pitch of the screw is shorter in the region of the inlet opening section facing the first screw pipe end than in the region of the inlet opening section facing the second screw pipe end.

17. Biogas plant, comprising a digester which can be sealed in a gas-tight manner and which has a solid matter feed opening in a gas discharge opening,
**characterised by** a screw conveyor as claimed in any one of the preceding claims, the outlet opening of which is connected directly to the solid matter feed opening or to a pipeline which is under internal digester pressure and which leads to the solid matter feed opening of the digester.

18. Method for feeding organic solids into a digester which is under internal pressure, said method comprising the steps of:
conveying the organic matter by means of a screw conveyor from an inlet opening to an outlet opening which is connected directly and in a gas-tight manner to the digester,
compacting the organic matter in the screw conveyor to prevent or reduce the passage of gas through the screw conveyor,
wherein the organic matter is preferably compacted by reducing the passage cross-section of the screw conveyor in the conveying direction and the passage cross-section is further expanded in the conveying direction prior to the reduction,
optionally separating fluid from the organic matter by means of a plurality of radial openings in the screw conveyor prior to compacting,
optionally flushing in a fluid after compacting.

## Revendications

1. Transporteur à vis servant à introduire des substances organiques dans un fermenteur à biogaz sous pression intérieure de gaz, lequel transporteur à vis comprend :
- un tube de vis (10) s'étendant dans une direction longitudinale, pourvu d'une ouverture d'entrée (30) pour des substances organiques dans la zone d'une première extrémité de tube de vis (11) et d'une ouverture de sortie (40) pour les substances organiques dans la zone d'une deuxième extrémité de tube de vis (12),
- une vis transporteuse (20) s'étendant dans la direction longitudinale et disposée dans le tube de vis, laquelle est logée de manière à pouvoir tourner et peut être amenée en rotation au moyen d'un entraînement de vis (50, 60),
- un raccord de fermenteur (40) servant à relier directement l'ouverture de sortie au fermenteur à biogaz sous pression de gaz,
- une portion de bourrage (80c) dans une zone entre l'ouverture d'entrée et l'ouverture de sortie servant à compacter les substances organiques afin de réduire un passage de gaz à travers le transporteur à vis,
**caractérisé en ce que** la portion de bourrage présente une zone d'élargissement (23a), dans laquelle la section transversale de passage entre la vis transporteuse et le tube de vis s'élargit dans le sens de transport, ainsi qu'une zone de rétrécissement (23b) disposée derrière la zone d'élargissement dans le sens de transport, dans laquelle la section transversale de passage entre la vis transporteuse et le tube de vis se réduit dans le sens de transport.

2. Transporteur à vis selon la revendication 1,
**caractérisé en ce que** la vis transporteuse
- présente une première section transversale de passage entre la vis transporteuse et le tube de vis dans une zone immédiatement au début de la portion de bourrage dans le sens de transport,
- présente une deuxième section transversale de passage agrandie par rapport à la première section transversale de passage derrière la première section transversale de passage, dans la zone de début de la portion de bourrage, et
- présente une troisième section transversale de passage réduite par rapport à la deuxième à la fin de la portion de bourrage.

3. Transporteur à vis selon la revendication 1 ou 2,
**caractérisé en ce que** la section transversale de passage s'élargit de manière étagée dans la zone de début dans la portion de bourrage dans le sens de transport et/ou se réduit en continu sur la majeure partie de la portion de bourrage.

4. Transporteur à vis selon l'une quelconque des revendications précédentes, **caractérisé en ce que** la section transversale de passage varie avant la portion de bourrage depuis une première section transversale de passage, qui présente une géométrie de section transversale **caractérisée par** une première dimension de passage libre, jusqu'à une deuxième section transversale de passage présentant une géométrie de section transversale, qui est **caractérisée par** une deuxième dimension de passage libre plus grande que la première dimension de passage libre, et varie par ailleurs également jusqu'à une troisième section transversale de passage présentant une géométrie de section transversale, qui est **caractérisée par** une troisième dimension de passage libre inférieure à la deuxième dimension de passage libre, la première et la troisième dimensions de passage libre coïncidant de préférence.

5. Transporteur à vis selon l'une quelconque des revendications précédentes, lequel transporteur à vis comprend :
- un tube de vis s'étendant dans une direction longitudinale pourvu d'une ouverture d'entrée pour des substances organiques dans la zone d'une première extrémité de tube de vis et d'une ouverture de sortie pour les substances organiques dans la zone d'une deuxième extrémité de tube de vis,
- une vis transporteuse s'étendant dans la direction longitudinale et disposée dans le tube de vis, laquelle est logée de manière à pouvoir tourner et peut être amenée en rotation au moyen d'un entraînement de vis,
- un raccord de fermenteur servant à relier directement l'ouverture de sortie au fermenteur à biogaz sous pression de gaz,
- une portion de bourrage servant à compacter les substances organiques afin de réduire un passage de gaz à travers le transporteur à vis,
**caractérisé par** une portion de séparation disposée, dans le sens de transport, avant la portion de bourrage, comprenant une pluralité d'ouvertures dans le tube de vis et un dispositif collecteur de liquide en dehors du tube de vis.

6. Transporteur à vis selon l'une quelconque des revendications précédentes, lequel transporteur à vis comprend :
- un tube de vis s'étendant dans une direction longitudinale pourvu d'une ouverture d'entrée pour des substances organiques dans la zone d'une première extrémité de tube de vis et d'une ouverture de sortie pour les substances organiques dans la zone d'une deuxième extrémité de tube de vis,
- une vis transporteuse s'étendant dans la direction longitudinale et disposée dans le tube de vis, laquelle est logée de manière à pouvoir tourner et peut être amenée en rotation au moyen d'un entraînement de vis,
- un raccord de fermenteur servant à relier directement l'ouverture de sortie au fermenteur à biogaz sous pression de gaz,
- une portion de bourrage servant à compacter les substances organiques afin de réduire un passage de gaz à travers le transporteur à vis,
**caractérisé par** une ouverture d'amenée de liquide de rinçage servant à amener un liquide de rinçage dans la section transversale de passage entre le tube de vis et la vis transporteuse dans la zone de la deuxième extrémité de tube de vis derrière la portion de bourrage dans le sens de transport.

7. Transporteur à vis selon la revendication 6,
**caractérisé en ce qu'**une ouverture d'entrée de rinçage est prévue au niveau du côté frontal du tube de vis et débouche dans une conduite de rinçage s'étendant de manière axiale dans la direction longitudinale du tube de vis, laquelle conduite de rinçage s'étend depuis la deuxième extrémité du tube de vis jusqu'à au moins une ouverture d'amenée de liquide de rinçage radiale, laquelle débouche dans la section transversale de passage entre la vis transporteuse et le tube de vis.

8. Transporteur à vis selon la revendication 6 ou 7,
**caractérisé en ce que** la vis transporteuse est réalisée dans la zone de la deuxième extrémité sous la forme d'un arbre creux pour la conduite du liquide de rinçage dans l'espace intérieur de la vis transporteuse.

9. Transporteur à vis selon l'une quelconque des revendications 7 à 9,
**caractérisé en ce que** la vis transporteuse est reçue de manière à pouvoir tourner, dans la zone de la deuxième extrémité, sur un manchon, lequel est fixé de manière solidaire en couple de rotation au niveau de tube de vis et s'étend de manière axiale dans un espace creux de la vis transporteuse.

10. Transporteur à vis selon l'une quelconque des revendications précédentes, lequel transporteur à vis comprend :
- un tube de vis s'étendant dans une direction longitudinale pourvu d'une ouverture d'entrée pour des substances organiques dans la zone d'une première extrémité de tube de vis et d'une ouverture de sortie pour les substances organiques dans la zone d'une deuxième extrémité de tube de vis,
- une vis transporteuse s'étendant dans la direction longitudinale et disposée dans le tube de vis, laquelle est logée de manière à pouvoir tourner et peut être amenée en rotation au moyen d'un entraînement de vis,
- un raccord de fermenteur servant à relier directement l'ouverture de sortie au fermenteur à biogaz sous pression de gaz,
- une portion de bourrage servant à compacter les substances organiques afin de réduire un passage de gaz à travers le transporteur à vis,
**caractérisé par** au moins une pale de vis dans la zone de la deuxième extrémité de tube de vis, laquelle est fixée de manière amovible sur la vis transporteuse.

11. Transporteur à vis selon la revendication 10,
**caractérisé par** une première pale de vis pouvant être montée sur le transporteur à vis et par une deuxième pale de vis pouvant être montée en variante par rapport à la première pale de vis, laquelle présente une section transversale, qui occupe, par rapport à la première pale de vis, une surface de section transversale plus grande de la section transversale de passage entre la vis transporteuse et le tube de vis.

12. Transporteur à vis selon l'une quelconque des revendications précédentes, lequel transporteur à vis comprend :
- un tube de vis s'étendant dans une direction longitudinale pourvu d'une ouverture d'entrée pour des substances organiques dans la zone d'une première extrémité de tube de vis et d'une ouverture de sortie pour les substances organiques dans la zone d'une deuxième extrémité de tube de vis ;
- une vis transporteuse s'étendant dans la direction longitudinale et disposée dans le tube de vis, laquelle est logée de manière à pouvoir tourner et peut être amenée en rotation au moyen d'un entraînement de vis,
- un raccord de fermenteur servant à relier directement l'ouverture de sortie au fermenteur à biogaz sous pression de gaz,
- une portion de bourrage servant à compacter les substances organiques afin de réduire un passage de gaz à travers le transporteur à vis,
**caractérisé en ce que** la paroi intérieure du tube de vis délimite une surface intérieure de section transversale non circulaire.

13. Transporteur à vis selon la revendication 12,
**caractérisé en ce que** la paroi intérieure du tube de vis délimite une surface intérieure de section transversale angulaire.

14. Transporteur à vis selon l'une quelconque des revendications précédentes, lequel transporteur à vis comprend :
- un tube de vis s'étendant dans une direction longitudinale pourvu d'une ouverture d'entrée et d'une ouverture de sortie dans la zone d'une deuxième extrémité de tube de vis,
- une vis transporteuse s'étendant dans la direction longitudinale et disposée dans le tube de vis, laquelle est logée de manière à pouvoir tourner et peut être amenée en rotation au moyen d'un entraînement de vis,
**caractérisé en ce que** l'ouverture d'entrée est réalisée sous la forme d'une ouverture radiale dans le tube de vis et s'étend sur une portion d'ouverture d'entrée dans la direction longitudinale du tube de vis, et **en ce que** la vis transporteuse présente, dans la zone de la portion d'ouverture d'entrée, un premier pas de vis et un deuxième pas de vis différent du premier.

15. Transporteur à vis selon la revendication 14,
**caractérisé en ce que** le pas de la vis varie en continu dans la zone de la portion d'ouverture d'entrée.

16. Transporteur à vis selon la revendication 14 ou 15,
**caractérisé en ce que** le pas de la vis dans la zone de la portion d'ouverture d'entrée dirigée vers de la première extrémité de tube de vis est plus petit que celui dans la zone de la portion d'ouverture d'entrée dirigée vers la deuxième extrémité de tube de vis.

17. Installation à biogaz, comprenant un fermenteur pouvant être fermé de manière étanche aux gaz et pourvu d'une ouverture d'amenée de substances solides d'une ouverture de conduite de gaz,
**caractérisée par** un transporteur à vis selon l'une quelconque des revendications précédentes, dont l'ouverture de sortie est reliée directement à l'ouverture d'amenée de matières solides ou à une conduite d'arrivée sous pression intérieure de fermenteur menant à l'ouverture d'amenée de matières solides du fermenteur.

18. Procédé servant à amener des substances solides organiques dans un fermenteur sous pression intérieure, comprenant les étapes suivantes consistant à :
- transporter les substances organiques au moyen d'un transporteur à vis depuis une ouverture d'entrée vers une ouverture de sortie reliée directement de manière étanche aux gaz au fermenteur,
- compacter les substances organiques dans le transporteur à vis afin d'empêcher ou de réduire le passage de gaz à travers le transporteur à vis,
- le compactage des substances organiques étant effectué par une réduction de la section transversale de passage du transporteur à vis dans le sens de transport et la section transversale de passage étant par ailleurs élargie dans le sens de transport avant la réduction,
- en option séparer le liquide des substances organiques par plusieurs ouvertures radiales dans le transporteur à vis avant le compactage,
- en option incorporer un liquide après le compactage.
